# EUROPEAN PATENT APPLICATION

(11) **EP 2 003 448 A1**
(43) Date of publication of application: **17.12.2008**
(21) Application number: 07730498.8
(22) Date of filing: 16.03.2007
(51) Int. Cl.: G01N 33/02

(54) **METHOD FOR EXTRACTING GLUTEN FROM PROCESSED AND UNPROCESSED FOODS BY MEANS OF HEAT BASED ON THE USE OF IONIC AND NON-IONIC DETERGENTS**

(30) Priority: 16.03.2006 ES 200600675
(71) Applicant: Consejo Superior de Investigaciones Cientificas (CSIC), 28006 Madrid (ES)
(72) Inventor: MÉNDEZ CORMÁN, Enrique, E-28049 Madrid (ES); HERNANDO ÁLVAREZ, Alberto, E-28049 Madrid (ES)
(74) Representative: Pons Arino, Angel
(86) International application number: PCT/ES2007/070058
(87) International publication number: WO 2007/104825

(57) **Abstract**

In general, this invention relates to the analysis of foods for coeliacs patients and, in particular, relates to a method for extracting gluten from foods, which is compatible with an enzyme-linked immunosorbent assay (ELISA) and also compatible with the Western blot technique, to compositions suitable for implementing said method, to kits that comprise said compositions and to a method for the quantification of the gluten present in a food.

## Description

### FIELD OF THE INVENTION

This invention relates, in general, to the analysis of foods for people with celiac disease and, in particular, it relates to a method for gluten extraction in foods, compatible with an enzyme-linked immunosorbent assay (ELISA) and also compatible with the Western blot technique, to a composition suitable for the implementation of said method, to kits which comprise said compositions and to a method for the quantification of the gluten present in a food.

### BACKGROUND OF THE INVENTION

Celiac Disease (CD) is a disease characterized by intolerance to the gluten prolamins of wheat, barley, rye and oats, which causes changes in the intestine which lead to malabsorption.

Gluten comprises a complex mixture of proteins, and whilst the toxic component or components of gluten which cause CD are not known, the provisional solution consists of eliminating all components of gluten in the diet of these patients. In fact, the only really effective treatment for people with celiac disease consists of following a strict gluten-free diet. For this reason, it is essential to precisely quantify the gluten content in foods intended to be consumed by people with celiac disease.

The routine protocol for gluten analysis in foods for people with celiac disease consists of extracting the gluten with a solution of reducing agents and the denaturing agent guanidine [García E. et al. Development of a general procedure for complete extraction of gliadins for heat processed and unheated foods. European Journal on Gastroenterology and Hepatology. Eur J Gastroenterol Hepatol 17. 5, 529-539 (2005)] followed by its quantification by ELISA [Valdés I., et al. Innovative approach to low level gluten determination in foods using a novel sandwich ELISA protocol. European Journal of Gastroenterology and Hepatolog. 15 (5) 465-74. (2003)].

However, one of the problems of the gluten extraction system which uses guanidine as denaturing agent stems from its application in the analysis of gluten using the Western blot technique. In short, this technique consists of (i) a first separation of the components of the gluten by electrophoresis in polyacrylamide gels in the presence of sodium dodecyl sulfate (SDS); (ii) an electro transfer of the proteins of the gel to a nitrocellulose or PVDF membrane] and (iii) an immunodetection of the proteins in the membrane by the use of antibodies.

The drawback of using guanidine as denaturing agent consists of the fact that it interferes with the SDS gel analysis system, so that it is necessary to eliminate it by several washes with water in a process of around 30-45 minutes duration. As a consequence of the washing, part of the omega (ω) gluten fraction, which represents between 20-35% of the gluten and part of the other alpha (α), beta (β) and gamma (γ) gluten fractions, are lost. The quantification of the concentration of the different gluten fractions contained in a sample using this technique is, therefore, less accurate. Consequently, a quantitative analysis by western blot of the gluten content in a sample using guanidine as denaturing agent is rather unreliable.

Therefore, there is the need to find alternative methods to extract the gluten contained in foods, which allows a reliable quantification thereof by conventional techniques such as the ELISA technique. Advantageously, said methods must permit the analysis of the different gluten fractions contained in said foods using Western blot.

### SUMMARY OF THE INVENTION

The invention tackles the problem of developing a method for the quantitative extraction of the gluten contained in foods, not only compatible with the ELISA technique currently existing for the quantification of gluten in foods but also with other conventional techniques, such as the Western blot technique.

The solution provided by this invention is based on the fact that the inventors have observed that the use of a composition which comprises a disulfide group-reducing agent and a detergent selected from sodium dodecyl sulfate (SDS) (ionic detergent) and any ester of polyoxyethylated sorbitan (Tween non-ionic detergent), as denaturing agents in a buffer with pH between 7 and 8, permits the quantitative analysis of the gluten contained in foods by an immunoassay such as ELISA, as well as the analysis of the gluten fractions contained in foods by Western blot.

Although the performance of gluten extraction in foods achieved by the method developed by this invention, using SDS or an ester of polyoxyethylated sorbitan as denaturing agent, is similar to that obtained by the conventional extraction system using guanidine as denaturing agent, i.e. both methods make it possible to extract practically the same quantity of gluten, the concentration of the different α, β, γ and ω gluten fractions which are obtained is different in each case, being greater when the method of the invention is used, since using the conventional system of gluten extraction with guanidine, the omega (ω) gluten fraction is lost during the washing process, as well as part of the other α, β and γ gluten fractions. Therefore, the quantification of the different gluten fractions by Western blot, in particular of the omega (ω) fraction, is more precise using the method developed by this invention.

Therefore, an object of this invention is constituted by a composition which comprises a disulfide group-reducing agent and one or several denaturing agents, in a buffer with pH between 7 and 8, wherein said denaturing agent is selected from SDS, an ester of polyoxyethylated sorbitan and their mixtures.

In another aspect, the present invention relates to a method for gluten extraction in a sample of a food which comprises extracting the gluten contained in said sample with an aqueous ethanol solution in the presence of a composition which contains a disulfide group-reducing agent and a denaturing agent, in a buffer with pH between 7 and 8, wherein said denaturing agent is selected from SDS, an ester of polyoxyethylated sorbitan and/or their mixtures.

An additional object of this invention is constituted by the use of said composition in the implementation of said method of gluten extraction in foods.

Another additional object of this invention is constituted by a method for the quantification of the gluten present in a sample of a food which comprises the prior extraction of the gluten contained in said sample using the aforementioned method of gluten extraction and, then, analysing the gluten content extracted using immunodetection methods such as ELISA, Western Blot, or alternatively, immunochromatography, immunoprecipitation, surface plasmon resonance (SPR), etc. Another additional object of this invention is constituted by a kit which comprises said composition useful for the implementation of said method of gluten extraction in foods, or their components separately. Said kit may be used to extract the gluten contained in a food as step prior to quantification by ELISA or analysis by Western blot of the gluten contained in a food.

### BRIEF DESCRIPTION OF THE FIGURES

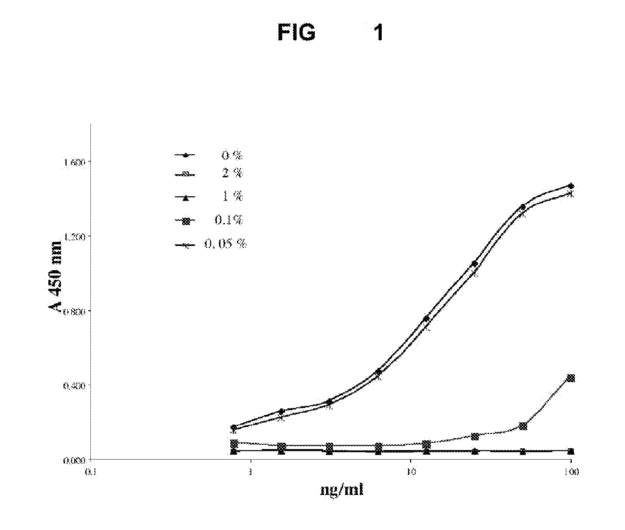

**Figure 1** represents standard curves at different known concentrations of gliadins whereto different quantities of SDS have been added to observe their effect in an ELISA plate coated with the R5 monoclonal antibody, supplied and owned by the Gluten Unit of the National Biotechnology Centre (CSIC). The final concentrations of SDS on the ELISA plate are 0%, 0.05%, 0.1%, 1% and 2%.

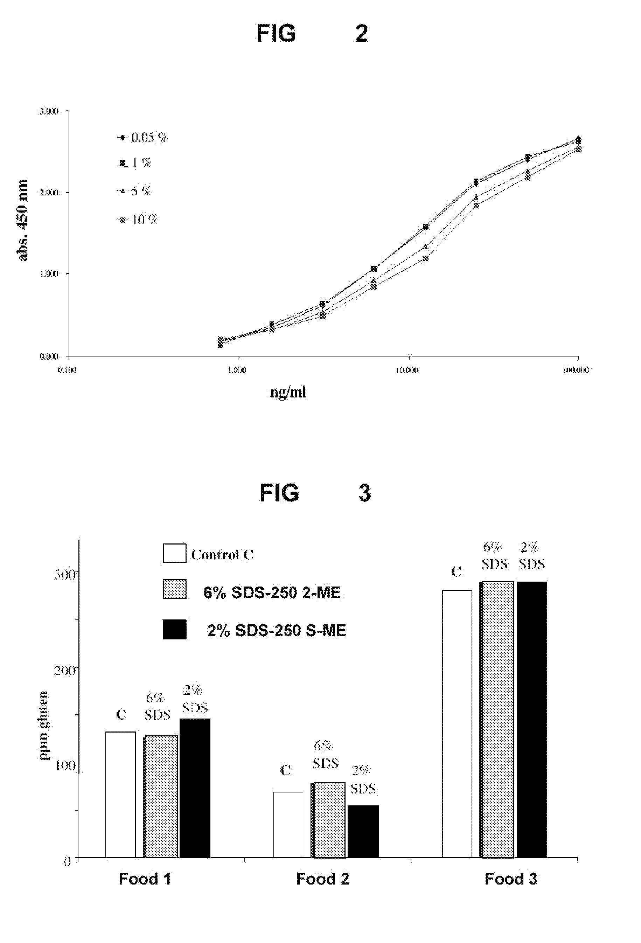

**Figure 2** represents standard curves at different known concentrations of gliadins whereto different quantities of Tween®-20 (P-1379 supplied by SIGMA) have been added to see their effect on the ELISA plate coated with the R5 monoclonal antibody, supplied and owned by the Gluten Unit of the National Biotechnology Centre (CSIC). The final concentrations of Tween®-20 on the ELISA plate are 0.05%, 1%, 5% and 10%.

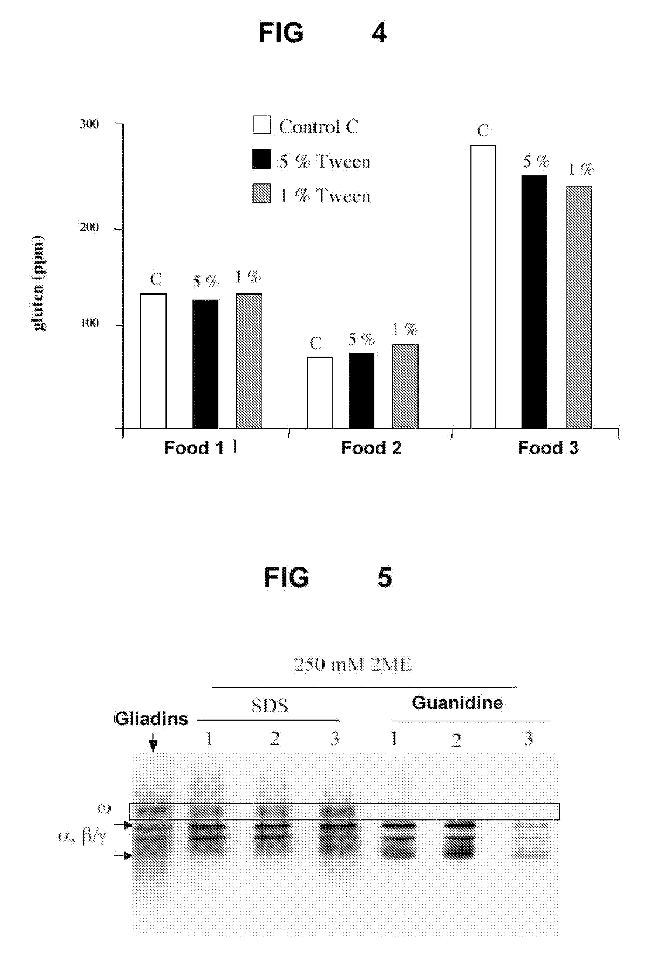

**Figure 3** shows a bar chart which represents gliadin recovery from samples (foods 1, 2 and 3) heat-treated and extracted with solutions which contain 250 mM 2-mercaptoethanol (2-ME) and 2% or 6% SDS. As an extraction control solution, guanidine/2-ME cocktail was used. The samples were incubated with these solutions at 50°C during 40 minutes and then they were subjected to an extraction with 60% ethanol during 60 minutes to extract the gliadins from the gluten. The quantification of the gluten in the extracts was performed using ELISA. **Figure 4** shows a bar chart which represents gliadin recovery from samples (foods 1, 2 and 3) heat-treated and extracted with solutions which contain 250 mM 2-mercaptoethanol (2-ME) and 1% or 5% Tween®-20 (P-1379 supplied by SIGMA). As control extraction solution, guanidine/2-ME cocktail was used. The samples were incubated with these solutions at 50°C during 40 minutes and then they were subjected to an extraction with 60% ethanol during 60 minutes to extract the gliadins from the gluten. The quantification of the gluten in the extracts was performed using ELISA. **Figure 5** shows a Western blot analysis of food gliadins (1, 2 and 3) heat-treated and extracted with 250 mM 2-ME containing 6% SDS or 2 M guanidine.

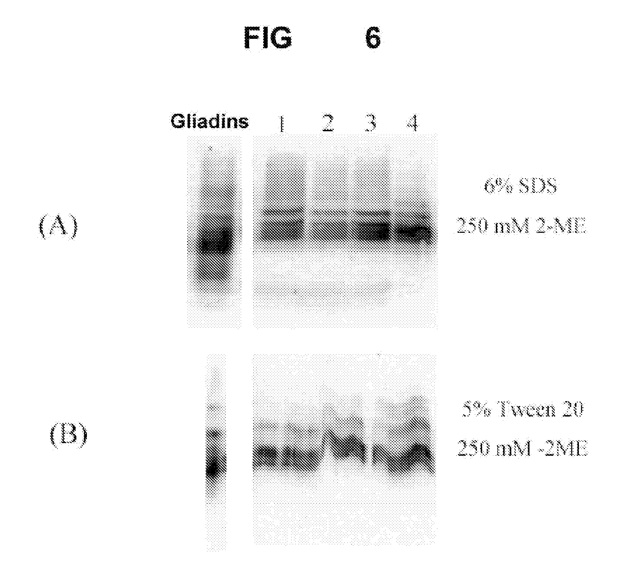

**Figure 6** shows a Western blot analysis of food gliadins (1, 2, 3 and 4) heat-treated and extracted with 250 mM 2-ME containing 6% SDS **(A)** or 5% Tween®-20 (P-1379 supplied by SIGMA) **(B).**

### DETAILED DESCRIPTION OF THE INVENTION

The invention relates, in general, to a composition, hereinafter composition of the invention, which comprises a disulfide group-reducing agent and a denaturing agent, in a buffer with pH between 7 and 8, wherein said denaturing agent is selected from SDS, an ester of polyoxyethylated sorbitan and their mixtures.

The disulfide group-reducing agent can be any compound capable of reducing disulfide groups known in the state of the art, for example, 2-mercaptoethanol (2-ME), dithiothreitol, etc. The composition of the invention may contain a single disulfide group-reducing agent or a mixture of two or more disulfide group-reducing agents. In a particular embodiment, the disulfide group-reducing agent of the composition of the invention is selected from 2-mercaptoethanol (2-ME), dithiothreitol and their mixtures. In a particular embodiment, the reducing agent used is 2-mercaptoethanol in a concentration between 50-500 mM, preferably between 150-400 mM. In a preferred particular embodiment, the concentration of 2-mercaptoethanol (2-ME) is 250 mM. In another particular embodiment, the reducing agent used is dithiothreitol (DTT); this can be used in a concentration between 0.1-30 mM, preferably between 0.5-10 mM, for example 5.0 mM.

The concentration of the disulfide group-reducing agent in the composition of the invention may vary within a wide range depending, among other things, on the disulfide group-reducing agent in question.

A denaturing agent is a compound with the capacity for opening the formation of proteins to make certain areas of the polypeptide chain more acceptable to external reagents and antibodies. The composition of the invention comprises a denaturing agent selected from the group formed by an ester of polyoxyethylated sorbitan, sodium dodecyl sulfate (SDS) and their mixtures. Although is possible to use several denaturing agents at the same time, it is not an advantageous option, since the presence of tween distorts the acrylamide gel. SDS, unlike the other denaturing agents, does not distort the acrylamide gel and makes it possible to observe all prolamin fractions in the gel without the need to perform washes, in order to avoid the loss of prolamin fractions caused by said washes. The concentration of the denaturing agent in the composition of the invention may vary within a wide range depending, among other things, on the denaturing agent in question. In a particular embodiment, the denaturing agent of the composition of the invention is sodium dodecyl sulfate (SDS) and the concentration of said compound in the composition of the invention is between 0.5-7% for example, 1-6%, preferably between 2-6%, for example: 2%, 2.5%, 3%, 3.5%, 4%, 4.5%, 5%, 5.5% or 6%. In a preferred particular embodiment the SDS concentration in the extraction composition of the invention is 6%. SDS, unlike the other denaturing agents, does not distort the acrylamide gel. In another particular embodiment, the denaturing agent is any ester of polyoxyethylated sorbitan, for example, but without being limited to, Tween®-20, Tween®-40 and Tween®-80; and their mixtures. The concentration of said compound in the composition of the invention may vary within a wide range. In a particular embodiment, it is between 1-5%, for example: 1%, 1.5%, 2%, 2.5%, 3%, 3.5%, 4%, 4.5% or 5%. In a more preferred embodiment, the ester of polyoxyethylated sorbitan is Tween®-20, which, unlike other denaturing agents, such as, for example, guanidine, allows a direct analysis of prolamins by Western blot without having to perform the step of washing with water and avoiding the loss of gluten fractions. In a preferred particular embodiment, the ester of polyoxyethylated sorbitan used in the extraction composition of the invention is Tween®-20 at a concentration between 1-5%, preferably 1%. As buffer it is possible to use any pH regulating solution which buffers at a pH value between 7 and 8, for example, saline solution with phosphates (PBS) with a pH between 7 and 8, Tris with pH between 7 and 8, etc.

The composition of the invention may be used as gluten extraction "cocktail" in foods, both heat processed and unheated. It has been surprising to verify that the use of the composition of the invention in gluten extraction not only permits a practically quantitative extraction of the gluten but it also does not adversely affect the quantification by ELISA of the gluten contained in a food. The extraction cocktail of the invention demonstrates being compatible with immunodetection techniques, such as ELISA, wherein the detection is carried out by the use of monoclonal or polyclonal antibodies. The use of the composition of the invention further presents the advantage of not losing any of the gluten fractions (α, β, γ, ω) during the extraction process since it does not require the execution of washes which drag part of the gluten fractions. In this way, the composition of the invention constitutes a useful composition to precisely quantify the gluten contained in a food. Additionally, although the final yield, i.e. the total concentration of gluten extracted with the method of the invention, is similar in comparison to the routine method which uses guanidine as denaturing agent, however, the routine method does not permit performing a precise quantification of the gluten content in the sample nor a reliable analysis of the different gluten fractions contained in the sample since, as has been previously stated, as a consequence of the washes a large part of the ω fraction is lost, which represents between 20-35 % of the gluten, and part of the other α, β and γ gluten fractions.

In another aspect, the invention relates to a method for gluten extraction in a sample of a food, hereinafter method of the invention, which comprises extracting the gluten contained in said sample with an aqueous ethanol solution in the presence of the composition of the invention. The resulting supernatant, which contains the gluten extracted, is removed for its subsequent analysis.

The ethanol concentration in the aqueous ethanol solution may vary within a wide range. In a particular embodiment, said aqueous ethanol solution has an ethanol content between 50% and 70%. In a preferred particular embodiment the ethanol content is 60%.

In another particular embodiment, the method of the invention, which comprises extracting the gluten with an aqueous ethanol solution in the presence of the composition of the invention, and separating the supernatant which contains the extracted gluten, is characterized in that before the extraction with said aqueous ethanol solution, the sample to assay is mixed with the composition of the invention, is incubated at a temperature between 24°C and 80°C, during a period of time between 15 and 40 minutes, and is cooled to ambient temperature (between 15°C and 25°C). In a preferred embodiment, the incubation of the sample with the composition of the invention is carried out at a temperature between 37°C and 50°C during a period of time between 15 and 40 minutes. Then, an aqueous ethanol solution is added, preferably a 60% aqueous ethanol solution, to said mixture and it is incubated at a temperature and during a suitable period of time, for example, at ambient temperature during a range between 10 and 60 minutes with stirring. In a preferred embodiment, the incubation of the mixture which contains the sample to assay, the composition of the invention and the aqueous ethanol solution, is carried out at ambient temperature in a range between 10 and 60 minutes.

Subsequently, the reaction mixture is centrifuged, the supernatant (which contains the gluten extracted) is removed and, finally, the gluten content is analysed using immunodetection methods such as ELISA, Western Blot, or alternatively immunochromatography, immunoprecipitation, surface plasmon resonance (SPR), etc., which are based on antibodies with specificity against gluten proteins, as is known in the state of the art and available by commercial means, for example, the "R5" antibody (Eur J Gastroenterol Hepatol 13:1-5. 2001) or the "Skerrit" antibody (J Assoc Off Anal Chem., 74: 257-64, 1991). The sample of foods to assay is prepared using conventional methods, which include, if applicable, the grinding of the sample which is deposited in a suitable receptacle, for example, a propylene tube. Then, the composition of the invention is added, the receptacle is closed and the resulting mixture is stirred. Advantageously, the receptacle which contains said mixture is sealed, for example, with sealing paper, in order to avoid the evaporation of said mixture by the action of heat.

Using the method of the invention it is managed to solubilize practically all the toxic gluten fractions contained in the foods, both heat processed and unheated. Although we do not want to be bound by any theory, it is thought that the composition of the invention acts by opening the formation of the gluten constituent proteins, in particular, of the toxic fraction thereof, and, as a consequence of this, their solubility in the extraction medium is favoured. Indeed, during the heat processing of the foods, the gluten fractions (α, β, γ and ω) are denatured, the disulfide bridges are broken, and consequently, insoluble aggregates are formed which cannot be extracted with 60% ethanol/water. The combined effect of the components of the composition of the invention means (i) that the insoluble aggregate can be extracted with an aqueous ethanol solution (50-70% ethanol/water) and the gluten content is analysed using immunodetection methods such as ELISA, Western Blot, or alternatively immunochromatography, immunoprecipitation, surface plasmon resonance (SPR), etc.,(ii) which opens the formation of the gluten molecules, the antigenic determinants remaining more exposed to antibodies, which produces an increase in the ELISA signal and permits the detection of all prolamin fractions and (iii) that the determination of the gluten concentration in a food is more precise when bearing in mind all fractions of gluten contained therein.

The method of the invention constitutes, therefore, a general method of gluten extraction from foods, especially designed for people with celiac disease, both heat processed and unheated, which solubilizes the toxic gluten fractions making it possible to quantitatively and reliably analyse the gluten in this type of foods.

The invention also provides a method for the ELISA quantification of the gluten present in a sample of a food which comprises extracting the gluten contained in said sample using the method of the invention and, then, quantifying the previously extracted gluten by ELISA. Furthermore, the invention provides a method for analysis, using Western blot, of the gluten fractions contained in a sample of food, which comprises extracting the gluten contained in said sample using the method of the invention and, then, analysing and/or quantifying by Western blot the previously extracted gluten. Both methods of quantification and analysis of gluten in a sample by ELISA or Western blot can be jointly combined in any order to analyse a sample of a food, whether first by ELISA and then by Western blot, or vice versa.

In another aspect, the invention relates to a kit which comprises the composition of the invention, or the components of said composition partially mixed or separated. This kit can be used to extract the gluten contained in a sample of a food, both heat processed and unheated, or, to extract the gluten as step prior to its (i) quantification by ELISA with the purpose of determining the gluten content of the sample of food assayed or to (ii) its analysis by other conventional immunodetection techniques, such as: Western blot, immunochromatography, immunoprecipitation, plasmon resonance, etc.; preferably using Western blot.

In a particular embodiment, the kit further comprises the other reagents necessary for the extraction of the gluten, i.e. the aqueous ethanol solution or its components separately, and/or the reagents necessary for the performance of an ELISA to quantify the gluten contained in a food, including antibodies with specificity against gluten proteins, as known in the state of the art and available through commercial means, for example, the "R5" antibody (Eur J Gastroenterol Hepatol 13:1-5. 2001) or the "Skerrit" antibody (J Assoc Off Anal Chem., 74: 257-64, 1991). Said reagents for the performance of the ELISA can be totally or partially mixed together or separated.

In another particular embodiment, the kit further comprises the reagents necessary for the performance of a Western blot to analyse the gluten fractions contained in a food, mixed totally or partially together or separated.

The following examples serve to illustrate the invention and must not be considered as limitative of the scope thereof.

### EXAMPLES OF EMBODIMENT

### EXAMPLE 1.- Recovery of gluten in foods by the use of SDS or an ester of polyoxyethylated sorbitan

This assay was performed to compare the efficacy of the method provided by the invention in gluten extraction in foods against the routine method which uses guanidine as denaturing agent.

### 1.1 Materials

The following foods and solutions have been used to execute this example:
- Food: gluten-free breads in corn base which have been contaminated with known quantities of gliadins (wheat prolamins) and subjected to high temperatures during the preparation process (180-220°C) and which are used as controls.
- 80% aqueous ethanol solution: it is prepared by mixing, for example in a test tube, 400 ml of ethanol (Scharlau Nv 121) and 100 ml of milli-Q water (18 MΩm resistivity) and it is stored in a glass bottle.
- Composition or SDS extraction cocktail: it is constituted by 250 mM 2-mercaptoethanol (2-ME), 6% SDS and 0.1x PBS, it is prepared, for example, weighing 1.2 grams of SDS Biochemical 44244 (molecular weight = 288.38 g/mol and adding 15-18 ml of a PBS solution (0.1x). Then, add 349 µl of 14.29 M 2-ME (Sigma M-6250), and flush to 20 ml with PBS solution (0.1x) and mix until achieving a final solution.
- Composition or Tween extraction cocktail: it is constituted by 250 mM 2-ME, 5% Tween®-20 and 0.1x PBS; it is prepared, for example, by adding 1.0 ml Tween®-20 (Sigma P-1379) Molecular weight = 14.29 g/mol and adding 15-18 ml of a PBS solution (0.1x). Then, add 349 µl of 2-ME, 14.29 M (Sigma M-6250), flush to 20 ml with PBS solution (0.1x) and mix until achieving a final solution.
- PBS solution (0.1x): it can be prepared by diluting 1:100 a PBS solution (10x). To do this, add 1 ml of PBS (10x) in a test tube and flush to 100 ml with milli-Q water (resistivity 18 MΩcm). The resulting solution must have a pH between 7 and 8. The PBS solution (10x) can be prepared by mixing and dissolving 80 g of NaCl, 2 g of KCl, 14.4 g of Na₂HPO₄ and 2.4 g of KH₂PO₄ in 900-950 ml of distilled water, in a 1 I beaker with magnetic stirring and flushing the resulting solution with distilled water until 1,000 ml in a volumetric flask. The solution produced is stored at ambient temperature.

### 5 1.2 Methods

### 1.2.1 Gluten extraction

The method according to the invention consists of carrying out gluten extraction using a 60% aqueous ethanol solution in the presence of a reducing agent (2-ME) and a denaturing agent (SDS or Tween®-20) and in a buffer with pH between 7 and 8.

To perform this method, the following steps are carried out:
a) weigh 0.125 g of the ground food sample and deposit it in a 10 ml propylene tube.
b) then, add 1.25 ml of a composition (extraction cocktail) constituted by 250 mM 2-ME, 6% SDS and PBS (0.1x) (SDS extraction cocktail) or 1.25 ml of a composition (extraction cocktail) constituted by 250 mM 2-ME, 5% Tween®-20 and PBS (0.1x) (Tween extraction cocktail) or, alternatively, a composition (extraction cocktail) constituted by 6% SDS and PBS (0.1x) (SDS extraction cocktail without 2-ME) or 1.25 ml of a composition (extraction cocktail) constituted by 5% Tween®-20 and PBS (0.1x) (Tween extraction cocktail without 2-ME). In this last case we want to check if the Tween or the SDS without the reducing agents are capable of extracting all the gluten. The tubes are closed with the screw/pressure cap and are sealed with sealing paper to avoid heat evaporation. The reaction mixture is stirred vigorously and incubated in a stove at a temperature between 27 and 80°C during a period of time between 15 and 40 minutes.
c) having finalized the incubation, the reaction mixture is left to cool to ambient temperature, and 3.75 ml of an 80% aqueous ethanol solution are added to the reaction mixture and it is incubated during a period of time between 15 and 40 minutes at ambient temperature in a rotary stirrer [Model LABINCO BV (position 2-3)];
d) next, the samples are centrifuged for 10 minutes at 3,500 rpm (2,500g) at ambient temperature (5810 Eppendorf Centrifuge) and the supernatant is transferred to clean 10 ml propylene tubes; and
e) then, the samples are analysed by ELISA or by Western blot.

### 1.2.2 Preparation of the sample for the analysis of gliadins extracted with SDS extraction cocktail.

a) Place 100 µl of the food extracted with SDS extraction cocktail in a 1500 µl tube;
b) dry in Speed-Vac *model Unijet II* at 37°C during the necessary time (minimum 40 minutes);
c) add 20 µl of electrophoresis buffer to the tube with the dry sample and close the tube;
d) stir vigorously for 1 minute, then boil at 100°C in a heating block for approximately 20 minutes and it is centrifuged for 1 minute at 5000 rpm; and
e) load the 20 µl of electrophoresis buffer which contain the sample in the gel.

### 1.2.3 Preparation of the sample for the analysis of gliadins extracted with Tween extraction cocktail

a) Place 100 µl 100 µl of the food extracted with Tween extraction cocktail in a 1500 µl tube;
b) dry in Speed-Vac *model Unijet II* at 37°C during the necessary time (minimum 40 minutes);
c) add 20 µl of electrophoresis buffer to the tube with the dry sample and close the tube;
d) stir vigorously for 1 minute, then boil at 100°C in a heating block for between 2 and 5 minutes and centrifuge for 1 minute at 5000 rpm; and
e) load the 20 µl of electrophoresis buffer which contain the sample in the gel.

### 1.2.4 Preparation of the sample for the analysis of gliadins extracted with guanidine/2-ME cocktail: Washes to eliminate the guanidine.

In first place, extract the gluten from the sample as described by Enrique García et al. Development of a general procedure for complete extraction of gliadins for heat processed and unheated foods. European Journal on Gastroenterology and Hepatology. Eur J Gastroenterol Hepatol 17. 5, 529-539 (2005) using an extraction cocktail composed of 250 mM 2-ME, 2M guanidine and PBS (0.1X) which contains: 13 mM NaCl, 0.3 mM KCl, 1 mM Na₂HPO₄ and 0.2 mM KH₂PO₄. Once the sample is extracted, it is prepared for its analysis as follows:
a) place 100 or 200 µl of the food extracted with guanidine cocktail in a 1,500 µl eppendorf;
b) dry in Speed-Vac *model Unijet II* at 37°C during the necessary time (minimum 40 minutes); whilst the sample is dried, approximately 3 ml of milli-Q water are placed to cool in a polypropylene tube within an ice box;
c) a first wash is then performed for which (i) 100 µl of cold water (0°C - 4°C) are added to the eppendorf tube on the sample; (ii) vigorously stir for approximately 5-10 seconds until the salts are dissolved; (iii) it is centrifuged for 1 minute at 13,200 rpm (it is necessary to pay attention to the orientation of the tube to later add the water with the dissolved salts on the opposite side to where the pellet is deposited); and (iv) the tube is carefully removed from the centrifuge and the water with the dissolved salts is added on the opposite side to where the pellet is deposited, which concludes the first wash;
d) then, perform a second wash, for which, steps (i)- (iv) of the first wash process are repeated; if 200 µl is dried, it is necessary to perform a third wash;
e) once the second wash has concluded (or the third if applicable), dry the pellet in the Speed-Vac *model Unijet II* at 37°C for the time necessary so that the sample dries (approximately 15 minutes);
f) add 20 µl of electrophoresis buffer to the tube with the dry sample;
g) stir vigorously for 1 minute, then it is incubated at 100°C between 2 and 5 minutes in a heating block and it is centrifuged for 1 minute at 5,000 rpm in the Eppendorf centrifuge (*model* 5415 *D);* and
h) load the 20 µl of electrophoresis buffer which contains the sample in the gel.

### 1.3 Results

### 1.3.1 Effect of the concentrations of the denaturing agents SDS and Tween in ELISA

The results of the gliadin curves containing different concentrations of SDS or Tween®-20 show that the gliadin curve containing a concentration of 0.05% SDS does not affect the ELISA and is equal to the gliadin curve without SDS. Likewise, the gliadin curve containing a concentration of 0.05, 1, 5 or 10% Tween®-20 does not affect the ELISA and is equal to the gliadin curve without Tween®-20 (see Figures 1 and 2).

On the other hand, the results of gliadin recovery from heat-treated samples and extracted with 250 mM 2-ME containing 2 and 6% SDS show that recovery with 6% SDS is practically quantitative and slightly greater to that produced with 2% SDS. Likewise, the results of gliadin recovery from samples heat-treated and extracted with 250 mM 2-ME containing 1 and 5% Tween show that recovery with 5% and with 1% is practically quantitative. These results are shown in Figures 3 and 4 and Tables 1 and 2.

**Table 1**

| **Gliadin recovery from samples heat-treated and extracted with SDS or Tween 20 detergents in the presence or absence of 250 mM 2-ME** | | | | | |
|---|---|---|---|---|---|
| | (250 mM 2ME) | | Detergents: (without 2ME) | | |
| Sample | Gliadins * (ppm) | SDS (6%) (%) | Tween 20 (5%) (%) | SDS (6%) (%) | Tween 20 (5%) + SDS (6%) (%) |
| 1 | 290 | 104 | 46 | 51 | 50 |
| 2 | 312 | 96 | 34 | 45 | 41 |
| 3 | 214 | 93 | 44 | 52 | 46 |
| 4 | 161 | 93 | 39 | 47 | 43 |
| 5 | 54 | 89 | 46 | 50 | 56 |
| 6 | 132 | 93 | | | |
| 7 | 54 | 89 | | | |
| 8 | 70 | 97 | | | |
| 9 | 280 | 99 | | | |
| 10 | 336 | 95 | | | |
| 11 | 70 | 103 | | | |
| 12 | 158 | 97 | | | |
| 13 | 18 | 122 | | | |

| | | | | | |
|---|---|---|---|---|---|
| **Samples incubated at 50°C/ 40 minutes followed by an extraction with 60% ethanol/60 minutes** *** Known gliadin values** **1-13 heat-treated samples (240°C)** **(%) gliadins recovered** | | | | | |

Table 1 shows the results of gliadin recovery from samples heat-treated and extracted with SDS or Tween®-20 in the presence or absence of 250 mM 2-ME. The results show that gliadin recovery in the absence of 2-ME with the denaturing agents SDS and Tween®-20, separately or combined, is only of the order of 50%.

Table 2 shows the results of gliadin recovery from samples heat-treated and extracted with 250 mM 2-ME/6% SDS (SDS extraction cocktail) at different incubation times and temperatures and at different extraction times with 60% ethanol.

**Table 2**

| **Gliadin recovery from samples heat-treated and extracted with 250 mM 2- ME/6% SDS at different incubation times and temperatures and at different extraction times with 60% ethanol.** | | | | | | | | | | | |
|---|---|---|---|---|---|---|---|---|---|---|---|
| **25 mM 2-ME / 6% SDS** | | | | | | | | | | | |
| Incubation time Incubation temperature Extraction time with 60% ethanol | | **40 min** | | | | **15 min** | | | | | |
| | | 50°C | 24°C | 37°C | | 50°C | | 60°C | | 80°C | |
| | | | | | | | | | | | |
| | | 60 | 10 | 10 | 60 | 10 | 60 | 10 min | 60 min | 10 | 60 min |
| | | min | min | min | min | min | min | | | min | |
| Sample | Gliadins * (ppm) | (%) | (%) | (%) | (%) | (%) | (%) | (%) | (%) | (%) | (%) |
| 1 | 290 | 104 | 96 | 105 | 98 | 108 | 112 | 108 | 110 | 109 | 108 |
| 2 | 132 | 93 | 91 | 89 | 95 | 89 | 95 | 89 | 92 | 88 | 86 |
| 3 | 312 | 96 | 80 | 88 | 90 | 98 | 103 | 100 | 102 | 101 | 100 |

| | | | | | | | | | | | |
|---|---|---|---|---|---|---|---|---|---|---|---|
| **Samples incubated at 50°C/ 40 minutes followed by extraction with 60% ethanol/60 minutes** *** Known gliadin values** **1-3 heat-treated samples (240°C)** **(%) gliadins recovered** | | | | | | | | | | | |

Therefore, the results of gliadin recovery from samples heat-treated and extracted with SDS or with Tween®-20 in the presence or absence of 250 mM 2-ME shows that gliadin recovery in the presence of 2-ME is quantitative with the two denaturing agents assayed, SDS and Tween®-20.

Likewise, the results of gliadin recovery from samples heat-treated and extracted with 250 mM 2-ME / 6% SDS (SDS extraction cocktail) at different incubation times and temperatures and at different extraction times with 60% ethanol show that there are no significant differences in gliadin recovery in the indicated conditions.

### 1.3.2 Effect of the denaturing agents SDS and Tween®-20 in Western blot

Despite the good performance of Tween®-20 to quantify the gluten, it has been verified that said agent can produce interference in gluten detection using the Western blot technique (Figure 6B).

The greatest disadvantage of the system with guanidine is that during the preparation process of the sample to be analysed in SDS gels, it is necessary to perform a wash with water to eliminate the guanidine. In this washing process, the ω gliadin fraction is also eliminated for which reason said fraction is not detected in the Western blot (Figure 5). In the method of the invention, a gluten extraction cocktail has been used which comprises SDS so that the samples are directly applied to the SDS gel without the need for washes with water, thus avoiding the ω gliadin fraction from being lost.

The use of the extraction cocktail with Tween®-20 has the drawback that the Tween®-20 reagent affects electrophoresis conditions in the SDS gels distorting the gliadin bands (Figure 6B) which does not occur when SDS is used and that the SDS reagent is the same used in the SDS-PAGE electrophoresis gels for which reason it does not affect the electrophoresis conditions nor distorts the gliadin bands (Figures 5 and 6A).

## Claims

1. A composition which comprises a disulfide group-reducing agent and a denaturing agent, in a buffer with pH between 7 and 8, wherein said denaturing agent is selected from SDS, an ester of polyoxyethylated sorbitan and their mixtures.

2. Composition according to claim 1, wherein said disulfide group-reducing agent is selected from 2-mercaptoethanol, dithiothreitol and their mixtures.

3. Composition according to either of claims 1 or 2, where said reducing agent is selected from 2-mercaptoethanol at a concentration between 150-350 mM and dithiothreitol at a concentration between 0.5-10 mM and where said denaturing agent is selected from SDS at a concentration between 2-6% and an ester of polyoxyethylated sorbitan at a concentration between 1-5%.

4. A method for gluten extraction in a sample of a food which comprises extracting the gluten contained in said sample with an aqueous ethanol solution in the presence of a composition according to any of claims 1 to 3.

5. Method according to claim 4, wherein said aqueous ethanol solution has an ethanol content between 50% and 70%.

6. Method according to either of claims 4 or 5, which comprises mixing the sample to assay with a composition according to any of claims 1 to 3, incubating the resulting mixture at a temperature between 30°C and 70°C, during a period of time between 10 and 60 minutes, cooling to ambient temperature, adding an aqueous ethanol solution and separating the supernatant which contains the extracted gluten.

7. Method according to claim 6, wherein the incubation of the sample with said composition is carried out at a temperature between 37°C and 50°C during a period of time between 15 and 40 minutes.

8. Method according to claim 6, wherein said aqueous ethanol solution is a 60% aqueous ethanol solution.

9. Method according to claim 6, which further comprises incubating the mixture contained in the sample to assay, the composition of claims 1 to 3 and the aqueous ethanol solution.

10. Method according to claim 9, wherein the incubation of the mixture contained in the sample to assay, the composition of claims 1 to 3 and the aqueous ethanol solution, is carried out at ambient temperature for 1 hour.

11. A method for the quantification of the gluten present in a sample of a food by an immunodetection technique which comprises extracting the gluten contained in said sample by a method according to any of claims 4 to 10 and, then, analysing the gluten extracted by said immunodetection technique.

12. A method for the quantification by ELISA of the gluten present in a sample of a food which comprises extracting the gluten contained in said sample by a method according to any of claims 4 to 10 and, then, quantifying the gluten extracted by ELISA.

13. A method for the quantification and analysis of the fractions by Western blot of the gluten present in a sample of a food which comprises extracting the gluten contained in said sample by a method according to any of claims 4 to 10 and, then, quantifying and analysing the gluten fractions extracted by Western blot.

14. Method for the quantification and analysis of the fractions which comprises the joint performance of the methods according to claims 12 and 13, whether first by ELISA and then by Western blot, or vice versa.

15. A kit which comprises a composition according to any of claims 1 to 3, or the components of said composition partially mixed or separated.

16. Kit according to claim 15, which further comprises an aqueous ethanol solution or the components of said aqueous ethanol solution separately.

17. Kit according to claim 15, which further comprises the reagents necessary for the performance of an immunodetection assay to analyse the gluten contained in a food, mixed totally or partially together or separated.

18. Kit according to claim 15, which further comprises the reagents necessary for the performance of an ELISA to quantify the gluten contained in a food, mixed totally or partially together or separated.

19. Kit according to claim 15, which further comprises the reagents necessary for the performance of a Western blot to analyse the gluten fractions contained in a food, mixed totally or partially together or separated.
